Europäisches Patentamt

European Patent Office

Office européen des brevets

(19)

(11) Veröffentlichungsnummer : **0 135 085**
**B1**

(12) **EUROPÄISCHE PATENTSCHRIFT**

(45) Veröffentlichungstag der Patentschrift :
10.09.86

(21) Anmeldenummer : 84109038.4

(22) Anmeldetag : 31.07.84

(51) Int. Cl.⁴ : **C 07 C 53/08, C 07 C 53/12,**
C 07 C 51/487,
C 07 C 51/42, C 07 C 51/44,
C 07 C 51/573,
C 07 C 69/16, C 07 C 67/60

(54) Verfahren zur Abtrennung von Jod und dessen Verbindungen aus den bei der Carbonylierung von Dimethylether, Methylacetat oder Methanol erhaltenen Carbonylierungsprodukten.

(30) Priorität : 18.08.83 DE 3329781

(43) Veröffentlichungstag der Anmeldung :
27.03.85 Patentblatt 85/13

(45) Bekanntmachung des Hinweises auf die Patenterteilung : 10.09.86 Patentblatt 86/37

(84) Benannte Vertragsstaaten :
AT BE CH DE FR GB IT LI NL

(56) Entgegenhaltungen :
DE-A- 1 092 897
DE-A- 3 049 007
US-A- 2 033 720

(73) Patentinhaber : HOECHST AKTIENGESELLSCHAFT
Postfach 80 03 20
D-6230 Frankfurt am Main 80 (DE)

(72) Erfinder : Erpenbach, Heinz, Dr.
Oberbuschweg 22
D-5000 Köln 50 (DE)
Erfinder : Gehrmann, Klaus, Dr.
Geschwister-Scholl-Strasse 32
D-5042 Erftstadt (DE)
Erfinder : Lork, Winfried
Siegfried-von-Westerburg-Strasse 14
D-5042 Erftstadt (DE)
Erfinder : Prinz, Peter
von-Geyr-Ring 104
D-5030 Hürth (DE)

EP 0 135 085 B1

## Beschreibung

Die Erfindung betrifft ein Verfahren zur Abtrennung von Jod und dessen Verbindungen aus den bei der Carbonylierung von Dimethylether und/oder Methylacetat und/oder Methanol erhaltenen Carbonylierungsprodukten Essigsäure und/oder Acetanhydrid und/oder Ethylidendiacetat.

Die DE-B-21 04 828 beschreibt ein Verfahren zur Entfernung geringer Halogenidverunreinigungen aus einer Essigsäure, welche vorzugsweise durch Reaktion eines Alkohols oder Olefins mit Kohlenmonoxid in Gegenwart eines Katalysatorsystems aus einer Edelmetall- und einer Halogenkomponente gewonnen wurde, durch Behandlung mit Kaliumpermanganat, Natriumpermanganat, Kaliumdichromat, Natriumdichromat, Chromtrioxid, Chromkaliumoxalat, Kaliumchlorchromat, Kaliumchlorat und/oder Kaliumchromat bei 16 bis 200 °C. Hierbei wird eine weniger als 500 ppb Halogenid, insbesondere Jodid, enthaltende Essigsäure vor oder auch während der Destillation mit einer der genannten Metallverbindungen in einer Menge bis zu 1,0 Gew %, bezogen auf die Essigsäure, in Berührung gebracht, wobei man eine Abtrennung der Halogenidverbindung zwischen 90 und 98 % erreicht. Zur Reinigung Acetanhydrid enthaltender Carbonylierungsprodukte ist dieses Verfahren weitaus weniger wirksam, da die genannten Oxydationsmittel auch mit Acetanhydrid reagieren und das Jodid daher nicht mehr binden können.

Die DE-A-22 56 510 beansprucht ein Verfahren zur Entfernung sehr geringer Mengen von Jod aus Essigsäure, bei dem in einem System aus zwei Destillationskolonnen die jodhaltigen Verbindungen durch Zusatz von Oxiden, Hydroxiden, Carbonaten, Bicarbonaten und Salzen schwacher organischer Säuren von Alkali- und Erdalkalimetallen sowie Gemischen der Alkali- oder Erdalkalimetallverbindungen mit Hypophosphoriger Säure je nach vorhandenem Jodgehalt bis zu einem Endgehalt von < 40 ppb bis < 5 ppb aus der eingesetzten Essigsäure entfernt werden können. Bei diesem Verfahren werden sowohl die Salze der Alkali- und Erdalkalimetalle als auch die Hypophosphorige Säure in Form wäßriger Lösungen eingesetzt. Das bedeutet, daß diese Reinigungsmethode zur Entfernung von verunreinigenden Jodverbindungen aus Essigsäureanhydrid nicht geeignet ist, da hierbei das Anhydrid einer Verseifung unterliegt.

Die DE-A-29 01 359 betrifft ein Verfahren zur Entfernung von Jod aus organischen Verbindungen, bei dem man die Jod enthaltenden organischen Verbindungen bei 50 bis 200 °C mit einem Oxidationsmittel behandelt und das Reaktionsgemisch gleichzeitig oder anschließend mit einem Adsorptionsmittel in Berührung bringt. Als Oxidationsmittel wird bevorzugt Sauerstoff oder Wasserstoffperoxid und als Adsorptionsmittel Aktivkohle verwendet. In den Beispielen wird als Oxidations-mittel Wasserstoffperoxid (20 %ig) angegeben. Die Entfernung des Jods gelingt zu 92,4 bis 99,5 %, wobei der Jodgehalt nur bis auf 40 ppm gesenkt wird, wa den Reinheitsansprüchen bei weitem nicht genügt. Auch hier ist von Nachteil, daß durch den Einsatz von Wasserstoffperoxid dem System ebenfalls Wasser zugeführt wird.

Die DE-A-31 07 731 beansprucht ein Verfahren zur Abtrennung organischer Jodverbindungen von Carbonylierungsprodukten des Methanols, Methylacetats und Dimethylethers durch Flüssigphasenextraktion mit einem nichtaromatischen Kohlenwasserstoff. Hierbei werden gemäß den Beispielen die Jodgehalte nur bis auf höchstens 100 ppb Jod herabgesetzt. Abgesehen von den zu hohen Jodwerten erfordert dieses Verfahren wegen der zusätzlichen Destillation des Extraktionsmittels einen wesentlich höheren Aufwand.

Nach der Lehre der DE-A-29 40 751 entfernt man als Verunreinigung vorhandene Jodverbindungen aus Carbonylierungsprodukten des Methylacetats durch Behandeln mit Cäsium-, Kalium- und/oder Natriumacetat, wobei man die entsprechenden Alkalimetalljodide erhält. Die Methyljodid-Abreicherung auf nur 2 ppm genügt nicht den Anforderungen an ein gereinigtes Carbonylierungsprodukt und dessen Weiterverarbeitung. Im übrigen wird durch die gaschromatographische Analyse auf Methyljodid der Gesamtjodgehalt im Carbonylierungsprodukt nicht vollständig erfaßt, so daß eine Aussage über die wirkliche Jodabtrennung schwer nachvollziehbar ist.

Die vorliegende Erfindung beschreibt nun ein Verfahren, bei dem sich die Entfernung jodhaltiger Substanzen nicht nur auf Alkyl- und Aryljodide oder andere leicht destillierbare jodhaltige Verbindungen beschränkt, sondern bei dem es gelingt, Jod in jeder Art von gebundener wie auch elementarer Form zu fixieren und von dem zu reinigenden Einsatzprodukt nahezu quantitativ abzutrennen. Als Beispiele entfernbarer Jodverbindungen seien Methyljodid, Butyljodid, Jodaceton, Jodbenzol, Acetyljodid, Benzoyljodid, Jodwasserstoff sowie gegebenenfalls substituierte Ammonium- und Phosphoniumjodide genannt.

Im einzelnen ist das Verfahren der Erfindung nunmehr dadurch gekennzeichnet, daß man, zur Herabsetzung der Menge des die Carbonylierungsprodukte verunreinigenden Gesamtjods auf Gehalte unterhalb 20 ppb Jod, die Carbonylierungsprodukte bei Temperaturen von 20 bis 250 °C mit Alkyl- oder Arylphosphinen oder heterocyclischen aromatischen Stickstoffverbindungen und mindestens einem der Metalle Kupfer, Silber, Zink oder Cadmium oder deren Verbindungen behandelt und von dem dadurch in nichtflüchtiger Form gebundenen Jod destillativ abtrennt.

Das Verfahren der Erfindung ist weiterhin bevorzugt und wahlweise dadurch gekennzeichnet, daß man

a) Carbonylierungsprodukte mi weniger als 500 ppm Gesamtjod einsetzt,

b) die Behandlung der Carbonylierungsprodukte bei Temperaturen von 100 bis 140 °C während 15 bis 120 Minuten vornimmt,

c) im Behandlungsgemisch das Gewichtsverhältnis Jod (-verbindung) zu Alkyl- und/oder Arylphosphin und/oder Stickstoffverbindung zu Metall (-verbindung) = 1 : (100-10 000) : (50-10 000) einstellt,

d) die Behandlung der Carbonylierungsprodukte in Gegenwart von Wasserstoff durchführt.

Zur Fixierung der jodhaltigen Verunreinigungen setzt man zweckmäßigerweise das erforderliche Gemisch aus z. B. Triphenylphosphin und Kupfer (II) acetat vor der letzten Reinigungsstufe zu, in der der hochsiedende Rückstand vom gewünschten Carbonylierungsprodukt entfernt wird. Die Ausschleusung der gebundenen jodhaltigen Verbindungen erfolgt unter destillativer Abtrennung des Reinproduktes über die Sumpffraktion gemeinsam mit dem hochsiedenden Rückstand. Verbrauchtes Behandlungsgemisch kann unter Zusatz von z. B. frischem Triphenylphosphin und Kupfer (II) acetat mit einem Teil des Sumpfabzuges zur nochmaligen Verwendung im Kreislauf geführt werden.

Selbstverständlich kann auch das aus der letzten Reinigungsstufe des gesamten Carbonylierungsprozesses abgezogene und bisher unbehandelte Produkt, welches < 500 ppm, vorzugsweise < 10 ppm, an verunreinigenden jodhaltigen Verbindungen enthält, erst jetzt einer Behandlung mit z. B. Triphenylphosphin und Kupfer (II) acetat zwecks Eliminierung dieser Verunreinigungen unterzogen werden.

Nach dem Verfahren der Erfindung kann der Gesamtjodgehalt im behandelten Carbonylierungsprodukt bis unter die Nachweisbarkeitsgrenze von < 5 ppb (= 5 Gewichtsteile Jod pro 1 Milliarde ($10^9$) Gewichtsteile Carbonylierungsprodukt) gesenkt werden.

Als Alkyl- bzw. Arylphosphine eignen sich besonders Tributylphosphin, Trioctylphosphin, Trilaurylphosphin und Triphenylphosphin, und als heterocyclische, aromatische Stickstoffverbindungen Pyridin, N-methylimidazol, 3-Picolin, 2,4-Lutidin, 3,4-Lutidin und Chinolin. Als Metallverbindungen werden bevorzugt die Acetate eingesetzt. Jedoch stellen auch die Oxide oder Acetylacetonate brauchbare Verbindungen dar, um im Gemisch mit Alkyl- und/oder Arylphosphin und/oder aromatischer Stickstoffverbindung die unerwünschten jodhaltigen Verbindungen zu fixieren. Im Falle des Einsatzes von Zink bietet sich die metallische Form besonders an.

Das Gewichtsverhältnis Jod(-verbindung) zu Alkyl- und/oder Arylphosphin und/oder Stickstoffverbindung zu Metall(-verbindung) kann in einem weiten Bereich variiert werden. Aus Gründen der Wirtschaftlichkeit sollte es jedoch Werte von 1 : 6 000 : 6 000 nicht wesentlich überschreiten.

Die Behandlung des Carbonylierungsproduktes kann unter Normaldruck, aber auch unter erhöhtem Druck erfolgen. Ein Zusatz geringer Mengen Wasserstoff beeinflußt das Ergebnis positiv.

Das Verfahren der Erfindung kann sowohl in diskontinuierlicher als auch kontinuierlicher Betriebsweise durchgeführt werden.

Die analytische Bestimmung des Gesamtjods erfolgt durch die jodkatalysierte Reaktion zwischen Arsen- und Cerionen mit photometrischer Endbestimmung des Cers.

Beispiel 1

100 g Essigsäureanhydrid, verunreinigt mit 1 ppm Gesamtjod, werden mit 0,5 g Triphenylphosphin und 0,3 g Kupfer (II) acetat versetzt und 1,5 Stunden bei 135 °C gerührt. Danach wird das Essigsäureanhydrid abdestilliert und analysiert. Der Gesamtjodgehalt liegt unter der Nachweisbarkeitsgrenze von < 0,005 ppm (< 5 ppb = < 5 pp $10^9$), was einer Jodentfernung von > 99,5 % entspricht.

Beispiel 2 (Vergleichsbeispiel)

100 g Essigsäureanhydrid, verunreinigt mit 1 ppm Gesamtjod, werden mit 0,5 g Triphenylphosphin versetzt und 1,5 Stunden bei 135 °C gerührt. Anschließend wird destilliert und in dem als Destillat übergehenden Essigsäureanhydrid ein Gesamtjodgehalt von 0,15 ppm gefunden. Die Jodabtrennung beträgt somit 85 %, bezogen auf den Jodeinsatz.

Beispiel 3 (Vergleichsbeispiel)

100 g Essigsäureanhydrid, verunreinigt mit 1 ppm Gesamtjod, werden mit 0,3 g Kupfer (II) acetat versetzt und 1,5 Stunden bei 135 °C gerührt. Danach wird das Essigsäureanhydrid abdestilliert und analysiert. Aus dem darin mit 0,15 ppm bestimmten Gesamtjodgehalt errechnet sich eine Jodabtrennung von 85 %, bezogen auf den Jodeinsatz.

Beispiel 4

100 g Essigsäure, verunreinigt mit 3 ppm elementarem Jod, werden 1 Stunde mit 1 g Triphenylphosphin und 0,5 g Kupfer (II) acetat am Rückfluß gekocht. Anschließend wird die Essigsäure destillativ vom Behandlungsgemisch getrennt und analysiert, wobei ein Jodgehalt von 9 ppb gefunden wird. Die Jodabtrennung beträgt demnach 99,7 %.

Beispiel 5

100 g Essigsäureanhydrid, verunreinigt mit 2,0 ppm Gesamtjod, 0,5 Triphenylphosphin und 0,2 g Zinkupulver werden in einen 250 ml-Rundkolben eingefüllt und 1,0 Stunde auf 135 °C erwärmt. Danach wird das so behandelte Essigsäureanhydrid vom Behandlungsgemisch abdestilliert und auf Gesamtjod untersucht. Dir Analyse ergibt einen Wert von < 5 ppb Gesamtjod, woraus sich die Jodabtrennung zu > 99,8 % errechnet.

## Beispiel 6

100 g Essigsäureanhydrid, verunreinigt mit 1,0 ppm Gesamtjod, 0,5 g Triphenylphosphin und 0,5 g Cadmium (II) acetat werden in einen 250 ml-Rundkolben eingefüllt und 2 Stunden bei 135 °C gerührt. Anschließend wird das so behandelte Essigsäureanhydrid abdestilliert und analysiert. Aus dem darin mit 10 ppb gefundenen Gesamtjodwert errechnet sich eine Jodabtrennung von 99,0 %, bezogen auf den Jodeinsatz.

## Beispiel 7

In einen 250 ml-Rundkolben werden 100 g Essigsäureanhydrid, das 40 ppm Gesamtjod enthält, 3,0 g Triphenylphosphin und 3,0 g Kupfer (II) acetat eingewogen und 1,5 Stunden bei 135 °C gerührt. Anschließend wird das so behandelte Essigsäureanhydrid destillativ von den bei der Behandlung fixierten Jodverbindungen und dem überschüssigen Behandlungsgemisch abgetrennt. Im abgetrennten Essigsäureanhydrid wird ein Gesamtjodgehalt von 7 ppb analysiert, woraus sich die Jodentfernung zu > 99,9 % errechnet.

## Beispiel 8

100 g Essigsäureanhydrid, verunreinigt mit 2,5 ppm Gesamtjod, 1,0 Triphenylphosphin und 1 g Kupferacetylacetonat werden in einem 250 ml-Rundkolben eingewogen und 0,5 Stunden lang bei 130 °C gerührt. Danach wird das behandelte Essigsäureanhydrid destillativ von den bei der Behandlung gebundenen Jodverbindungen abgetrennt und analysiert. Der Gesamtjodgehalt liegt unter der Nachweisbarkeitsgrenze von < 5 ppb, was einer Jodabtrennung von > 99,8 % entspricht.

## Beispiel 9

100 g Essigsäureanhydrid, verunreinigt mit 70 ppm Gesamtjod, werden mit 3 g Triphenylphosphin und 1,5 g Zinkpulver 1,5 Stunden bei 135 °C gerührt. Danach wird das Essigsäureanhydrid abdestilliert und analysiert. Es wird ein Gesamtjodgehalt von 15 ppb ermittelt, woraus sich die Jodabtrennung zu > 99,9 % errechnet.

## Beispiel 10

100 g Essigsäureanhydrid, verunreinigt mit 3 ppm Gesamtjod, werden mit 0,7 g Triphenylphosphin und 0,5 g Silberacetat 0,75 Stunden bei 135 °C gerührt. Bei der destillativen Abtrennung des Essigsäureanhydrids erhält man ein Destillat mit einem Gesamtjodgehalt von 7 ppb. Die Jodabtrennung beträgt 99,8 %.

## Beispiel 11

100 g Essigsäureanhydrid, verunreinigt mit 2 ppm Gesamtjod, 0,5 g Triphenylphosphin und 0,2 g Kupfer(II)acetat werden in einen 250-ml-Rundkolben eingewogen und 2 Stunden auf 110 °C erwärmt. Anschließend wird das behandelte Essigsäureanhydrid vom Behandlungsgemisch durch Destillation abgetrennt und auf Gesamtjod untersucht. Die Analyse ergibt einen Wert von 5 ppb Gesamtjod, woraus sich eine Jodabtrennung von 99,8 % ergibt.

## Beispiel 12

100 g Essigsäureanhydrid, verunreinigt mit 2,5 ppm Gesamtjod, werden mit 0,3 g N-Methylimidazol und 0,5 g Kupfer(II)acetat gemischt und 2,0 Stunden bei 135 °C gerührt. Anschließend wird das Anhydrid abdestilliert und analysiert. Es wird ein Gesamtjodgehalt von 15 ppb gefunden, woraus sich eine Jodabtrennung von 99,4 % errechnet.

## Beispiel 13

100 g Essigsäureanhydrid, verunreinigt mit 2,5 ppm Gesamtjod, 0,75 g 2,4 Lutidin und 0,5 g Zinkpulver werden in einen 250-ml-Rundkolben eingewogen und 1,5 Stunden bei 135 °C gerührt. Danach wird das Essigsäureanhydrid abdestilliert und analysiert. Es wird ein Gesamtjodgehalt von 10 ppb ermittelt, woraus sich die Jodabtrennung zu 99,6 % ergibt.

## Beispiel 14

100 g Essigsäureanhydrid, verunreinigt mit 1 ppm Gesamtjod, werden mit 0,3 g Tributylphosphin und 0,3 g Kupfer(II)acetat versetzt und 2,0 Stunden bei 125 °C gerührt. Danach wird das Essigsäureanhydrid abdestilliert und analysiert. Aus dem darin mit 10 ppb bestimmten Gesamtjodgehalt errechnet sich eine Jodabtrennung von 99,0 %.

## Beispiel 15

100 g Essigsäureanhydrid, verunreinigt mit 2 ppm Gesamtjod, werden mit 0,3 g Tributylphosphin und 0,3 g Kupfer(II)acetat versetzt und 2 Stunden bei 125 °C gerührt. Während dieser Zeit werden 2 Normliter/h Wasserstoff durch die Mischung geleitet. Danach wird das Essigsäureanhydrid abdestilliert und analysiert. Es wird ein Gesamtjodgehalt von < 5 ppb ermittelt, woraus sich eine Jodabtrennung von > 99,8 % errechnet.

## Patentansprüche

1. Verfahren zur Abtrennung von Jod und dessen Verbindungen aus den bei der Carbonylierung von Dimethylether, Methylacetat oder Methanol erhaltenen Carbonylierungsprodukten Essigsäure, Acetanhydrid oder Ethylidendiacetat, dadurch gekennzeichnet, daß man, zur Herabsetzung der Menge des die Carbonylierungsprodukte verunreinigenden Gesamtjods auf Gehalte unterhalb 20 ppb Jod, die Carbonylierungsprodukte bei Temperaturen von 20 bis 250 °C mit Alkyl- oder Arylphosphinen oder heterocyclischen aromatischen Stickstoffverbindungen und mindestens einem der Metalle Kupfer, Silber, Zink oder Cadmium oder deren Verbindungen behandelt und von dem dadurch in nichtflüchtiger Form gebundenen Jod destillativ abtrennt.

2. Verfahren nach Anspruch 1, dadurch gekennzeichnet, daß man Carbonylierungsprodukte mit weniger als 500 ppm Gesamtjod einsetzt.

3. Verfahren nach einem der vorhergehenden Ansprüche, dadurch gekennzeichnet, daß man die Behandlung der Carbonylierungsprodukte bei Temperaturen von 100 bis 140 °C während 15 bis 120 min vornimmt.

4. Verfahren nach einem der vorhergehenden Ansprüche, dadurch gekennzeichnet, daß man im Behandlungsgemisch ein Gewichtsverhältnis Jod (-verbindung) zu Alkyl- oder Arylphosphin oder Stickstoff-Verbindung zu Metall (-verbindung) = 1 : (100-10 000) : (50-10 000) einstellt.

5. Verfahren nach einem der vorhergehenden Ansprüche, dadurch gekennzeichnet, daß man die Behandlung der Carbonylierungsprodukte in Gegenwart von Wasserstoff durchführt.

## Claims

1. Process for separating iodine and its compounds from the carbonylation products acetic acid, acetic anhydride or ethylidene diacetate obtained by subjecting dimethylether, methyl acetate or methanol to a carbonylation reaction which comprises : reducing the quantity of total iodine contaminating the carbonylation products to less than 20 ppb iodine by treating the carbonylation products at temperatures of 20 to 250 °C with alkyl or aryl phosphines or heterocyclic aromatic nitrogen compounds and at least one of the metals copper, silver, zinc or cadmium or their compounds and distillatively separating them from the iodine so fixed in non volatile form.

2. Process as claimed in claim 1, wherein carbonylation products containing less than 500 ppm total iodine are used.

3. Process as claimed in any of the preceding claims, wherein the carbonylation products are treated at temperatures of 100 to 140 °C over a period of 15 to 120 minutes.

4. Process as claimed in any of the preceding claims, wherein a ratio by weight of iodine(-compound) to alkyl or aryl phosphine or nitrogen compound to metal(-compound) of 1 : (100-10 000) : (50-10 000) is established in the mixture to be treated.

5. Process as claimed in any of the preceding claims, wherein the carbonylation products are treated in the presence of hydrogen.

## Revendications

1. Procédé de séparation de l'iode et de ses composés des produits de carbonylation acide acétique, anhydride acétique ou diacétate d'éthylidène obtenus dans la carbonylation de l'éther diméthylique, de l'acétate de méthyle ou du méthanol, caractérisé en ce que, pour réduire la quantité de l'iode total contaminant les produits de carbonylation à une teneur inférieure à 20 ppb, on traite les produits de carbonylation à des températures de 20-250 °C par des alkylphosphines ou arylphosphines ou des composés azotés aromatiques hétérocycliques et au moins l'un des métaux cuivre, argent, zinc et cadmium ou leurs composés et on les sépare par distillation de l'iode ainsi lié sous forme non volatile.

2. Procédé selon la revendication 1, caractérisé en ce que l'on traite des produits de carbonylation contenant une quantité d'iode total inférieure à 500 ppm.

3. Procédé selon l'une des revendications précédentes, caractérisé en ce que l'on traite les produits de carbonylation pendant 15 à 120 min à des températures de 100 à 140 °C.

4. Procédé selon l'une des revendications précédentes, caractérisé en ce que l'on établit dans le mélange à traiter une proportion pondérale (composé d')iode/alkylphosphine ou arylphosphine ou composé azoté/(composé de) métal égale à 1 : (100-10 000) : (50-10 000).

5. Procédé selon l'une des revendications précédentes, caractérisé en ce que l'on traite les produits de carbonylation en présence d'hydrogène.